(19) Europäisches Patentamt
European Patent Office
Office européen des brevets

(11) **EP 2 313 768 B1**

(12) **EUROPEAN PATENT SPECIFICATION**

(45) Date of publication and mention of the grant of the patent:
**23.01.2019 Bulletin 2019/04**

(21) Application number: **09800018.5**

(22) Date of filing: **13.03.2009**

(51) Int Cl.:
*G01N 33/44* (2006.01)    *G01N 15/02* (2006.01)
*G01N 15/06* (2006.01)    *G01N 30/00* (2006.01)
*G01N 35/08* (2006.01)

(86) International application number:
**PCT/EP2009/053013**

(87) International publication number:
**WO 2010/009907 (28.01.2010 Gazette 2010/04)**

(54) **DETERMINATION OF THE HYDRODYNAMIC RADII AND/OR CONTENT OF CONSTITUENTS OF A MIXTURE BY ANALYSIS OF THE TAYLOR DISPERSION OF THE MIXTURE IN A CAPILLARY TUBE**

BESTIMMUNG DER HYDROMECHANISCHEN RADIEN UND/ODER DES INHALTS AN BESTANDTEILEN EINER MISCHUNG DURCH ANALYSE DER TAYLOR-DISPERSION DER MISCHUNG IN EINEM KAPILLARRÖHRCHEN

DÉTERMINATION DES RAYONS HYDRODYNAMIQUE ET/OU DU CONTENU DE COMPOSANTS D'UN MÉLANGE PAR ANALYSE DE LA DISPERSION TAYLOR DU MÉLANGE DANS UN TUBE CAPILLAIRE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **21.07.2008 EP 08305409**

(43) Date of publication of application:
**27.04.2011 Bulletin 2011/17**

(73) Proprietors:
• **Centre National de la Recherche Scientifique (C.N.R.S.)**
**75016 Paris (FR)**
• **Université Montpellier 2**
**34095 Montpellier Cedex 5 (FR)**

(72) Inventors:
• **COTTET, Hervé**
**34920 Le Cres (FR)**
• **MATMOUR, Rachid**
**63100 Clermont-Ferrand (FR)**
• **BIRON, Jean Philippe**
**34380 St martin de Londres (FR)**
• **MARTIN, Michel**
**F-91220 Le Plessis-Pâté (FR)**

(74) Representative: **Lavoix**
**2, place d'Estienne d'Orves**
**75441 Paris Cedex 09 (FR)**

(56) References cited:
**WO-A-2008/031958    US-A1- 2003 234 356**

• **COTTET H ET AL: "Taylor dispersion analysis of mixtures" ANALYTICAL CHEMISTRY, vol. 79, no. 23, 1 December 2007 (2007-12-01), pages 9066-9073, XP002512113 ISSN: 0003-2700**
• **KELLY B ET AL: "Using Taylor dispersion profiles to characterize polymer molecular weight distributions" PCCP - PHYSICAL CHEMISTRY CHEMICAL PHYSICS, vol. 6, no. 24, 21 December 2004 (2004-12-21), pages 5523-5530, XP002512114 ISSN: 1463-9076**
• **CALLENDAR R ET AL: "Diffusion coefficients for binary, ternary, and polydisperse solutions from peak-width analysis of Taylor dispersion profiles" JOURNAL OF SOLUTION CHEMISTRY, vol. 35, no. 3, March 2006 (2006-03), pages 353-379, XP002512115 ISSN: 0095-9782**

- COTTET H ET AL: "Determination of dendrigraft poly-L-lysine diffusion coefficients by Taylor dispersion analysis" BIOMACROMOLECULES, vol. 8, no. 10, October 2007 (2007-10), pages 3235-3243, XP002512116 ISSN: 1525-7797 cited in the application

- Upma Sharma ET AL: "Diffusivity of Solutes Measured in Glass Capillaries Using Taylor's Analysis of Dispersion and a Commercial CE Instrument", Analytical Chemistry, vol. 77, no. 3, 1 February 2005 (2005-02-01), pages 806-813, XP055113297, ISSN: 0003-2700, DOI: 10.1021/ac048846z

**Description**

The instant invention relates to a method for analysing specific complex mixtures, which takes profit of the phenomenon of Taylor dispersion which takes place when species are mobilized in a capillary tube under hydrodynamic flow. The method of the invention, which allows to easily establish both the dimensions and the contents of the constituents of the mixture, is especially suitable for analysing a polymerization medium comprising a mixture including monomers and corresponding polymers.

In the field of analysis, a recurrent problem is the study of complex mixtures. Analysis of such mixtures often implies to separate the different constituents of the mixture (by the way of a chromatography, for example) before analysing the so separated constituents. The separation of the constituents may reveal to be difficult (and in certain cases almost impossible) to be carried out. Besides, such a separation is time consuming and further tends to affect the efficiency of the analysis.

Hence, analysis methods allowing obtaining direct information about the individual constituents of a mixture are of great interest. There are however very few analysis methods allowing such information to be obtained.

Article COTTET H et al, "Taylor dispersion analysis of mixtures", NALYTICAL CHEMISTRY, vol. 79, no.23, 1st December 2002, pages 9066-9073, XP00251 2113, tSSN 0003-2700, described a method of analysing mixtures of a monodisperse phthalate and a polydisperse poly(styrenesulfonate) in various proportions using TDA with an absorption detector, capable of detecting both species simultaneously. A comparison of calculated and experimental traces allowed the contents of the two species to be established. Moreover, experimental diffusion coefficients could be compared with theoretical values using an equation that determined the average diffusion coefficient from a weighted sum of individual values. It follows from the Stokes Einstein relationship that the diffusion coefficient is inversely proportional to the hydrodynamic radius. So, calculating one parameter automatically allows the other to be inferred.

Article KELLY B et al, "Using Taylor dispersion profiles to characterize polymer molecular weight distributions" PCCP - PHYSICAL CHEMISTRY CHEMICAL PHYSICS, vol. 6, no. 24,21st December 2004, pages 5523-5530, XP00251 21 1 4, ISSN 1 463-9076 also described a method of analysing a mixture by TDA, where the mixture comprises ethylene glycol as a first, monodisperse species and five different ethylene glycol oligomers as a second, polydisperse species. The polydisperse profiles are then analysed by fitting sets of monodisperse profiles with diffusion coefficients to the measured detector voltages.

Article CALLENDAR R et al, "Diffusion coefficients for binary, ternary, and polydisperse solutions from peak-width analysis of Taylor dispersion profiles" JOURNAL OF SOLUTION CHEMISTRY, vol. 35, no. 3, March 2006, pages 353-379, XP00251 21 15, ISSN 0095-9782 described the use of TDA to analyse mixtures of monodisperse and polydisperse species. The monodisperse species comprised ethylene glycol whereas the polydisperse species comprised ethylene glycol oligomers. A least squares procedure is used to determine molecular weight distributions and thus distributions of contributions of each glycol in the mixture. In this way, the content and the mean hydrodynamic radius, obtained from the dispersion coefficient via the Stokes Einstein relationship, are obtained for the polydisperse species and for the monodisperse species.

Especially, there is a need for a method which would allow obtaining information about the content of polymerization mixtures resulting from the polymerization of monomers or mixtures of polymers, especially about the remaining content of monomers (which reflects the degree of conversion) and the size of the formed polymers.

The instant invention aims at providing a new analysis method which allows to obtain direct information about the content and the size of the constituent of a mixture without having to preliminarily separate the constituents of the mixture.

In this connection, the invention especially aims at providing a method suitable for directly and simply analysing a polymerization medium including monomers and corresponding polymers and allowing to easily determine both the remaining content of monomers in the medium and the size of the formed polymers.

To this end, the instant invention provides a new method of analysis which makes use of the phenomenon of Taylor dispersion in a capillary tube. This new method is suitable for analysing specific mixtures, namely of the type including at least two kind of species which may be distinguished by a distinct response on a detection device.

More precisely, the invention relates to a method for analysing a mixture M according to claim 1.

The method of the invention implements a Taylor dispersion of the species (i) and (ii) of the mixture M inside a capillary tube (step (A)) and a measurement of a signal reflecting the Taylor dispersion obtained in that way (step (B)), these steps being specifically followed by an analysis of the obtained signal (step (C)).

In practice, the signal of step (B) is generally obtained in the form of a diagram reflecting the evolution in time of the intensity detected by the detector placed in the region of the outlet of the capillary tube. This diagram, which is analogous to the chromatograms obtained with chromatographic methods, will be referred hereinafter as a "taylorgram", since it reflects the Taylor dispersion.

The Taylor dispersion which is used in the method of the invention is a well known phenomenon, which occurs when a species (especially in solution or dispersion) is forced to move in a hollow tube having a small internal diameter, by inducing a hydrodynamic flow in the tube. The hydrodynamic flow brings about a dispersive velocity profile within the tube, generally parabolic (of the Poiseuille profile type), the molecules or particles of a species which are closest to the wall of the tube having a displacement velocity which is almost zero, this velocity increasing as these molecules or particles move closer to the axis, with a maximum velocity for those which are located at the centre of the tube. It results a dispersive profile of the species at the outlet of the tube, reflected by a broadened peak on a taylorgram measured at the outlet of the tube. In this connection, reference may especially be made to the articles of G. Taylor, in Proc. Roy. Soc., A, 219, 186-203 (1953) and of R. Aris, in Proc. Roy. Soc. Lond. A., 235, 67-77 (1956)

It is well known from the prior art to make use of the Taylor dispersion for establishing the hydrodynamic radius of a single species. As a matter of fact, the hydrodynamic radius of a given species is related to the diffusion coefficient of this species, and said diffusion induces a more or less widening of the peak of the taylorgram (generally the lower the diffusion coefficient, the higher the broadening).

The analysis of the broadening for a single (monodisperse) species (referred to as "Taylor Dispersion Analysis") allows direct establishment of hydrodynamic radius $R_h$ by using the following relationship:

$$H = \frac{kT}{3\pi\eta R_h u} + \frac{\pi\eta R_h d_c^2 u}{16 kT},$$

wherein: u is the linear displacement velocity of the species which is subject to the hydrodynamic flow of the carrier liquid; $d_c$ is the inner diameter of the tube used; T the absolute temperature; k is the Boltzmann constant; $\eta$ is the viscosity of the carrier liquid in which the species is dispersed ; and H is the plate height

$$H = \frac{l_s \sigma_t^2}{t_d^2},$$

of the species (directly linked to the width of the detected peak), calculated as follows: wherein: $l_s$ is the length travelled by the solute in the tube up to the detector; $t_d$ is the mean detection time of the peak; $\sigma_t^2$ is the time variance of the detected peak.

$$H = \frac{l_s \delta^2}{5,54.t_d^2},$$

This relationship being written, for the specific example of a Gaussian peak: where $\delta$ is the width of the peak at half-height.

As known in classical Taylor experiments, this expression of H can be modified to take into account some corrections due to the finite length of the injection plug and the pressure ramp. These corrections are described in the article by H. Cottet, M. Martin, A. Papillaud, E. Souaïd, H. Collet, A. Commeyras, Biomacromolecules, 2007, 8, 3235-3243. The average hydrodynamic radius of the solutes can be then derived from one taylorgram obtained at a given mobilization

$$R_h = \frac{2kT}{3\pi\eta u} \frac{1}{H \pm \sqrt{H^2 - \frac{d_c^2}{12}}}.$$

velocity u using the corrected H value according to the following equation:

This equation provides two values of the hydrodynamic radius. One of them can generally be discarded as being non-physical. If this is not the case, at least one additional analysis, performed at a different flow velocity (or mobilizing pressure) is required to solve this indetermination problem. The common value is then the correct one.

Diffusion coefficient D and the hydrodynamic radius $R_h$ can be also obtained from the slope of the linear part of the $H = f(u)$ curve, this slope being equal to: $\pi\eta R_h d_c^2 /(16kT)$.

Another technique to get rid of this corrections in a certain extent, is to use a detection device having two points of measurements, whether two detectors along the capillary tube or a loop in the capillary tube effecting the mixture to pass twice in front of the same detector, $l_s$ being then the distance between the two points of detection along the capillary tube. This was described in the article by A. J. S. Chapman and D. M. Goodhall, Chromatography Today, Vol.1, June 2008, 22-24.

The Taylor Dispersion Analysis described hereinabove is not suitable for analysing media comprising a mixture of species. In fact, a direct Taylor dispersion analysis of a mixture conducted as described above leads to a global signal which reflects the global properties of the mixture, namely the average size of the whole species present in the mixture. This global information does not provide any indication on the specific properties of each of the species of the mixture, especially when the mixture contains monomers and corresponding polymers.

Unexpectedly, in the scope of the instant invention, the inventors have now found that taylorgrams which are obtained for most of the mixtures can actually be interpreted so as to establish respective contributions of each species which are part of the mixture. More precisely, the inventors have surprisingly evidenced that a deconvolution of the taylorgram of a mixture is possible when the mixture contains a first species of predetermined nature, known as having a monodisperse distribution (defined molecule or defined macromolecule, for example) and a second polydisperse species having a response coefficient different from the response coefficient of the first species on a detection device.

As intended in the instant description, the expression "response coefficient" of a species on a given detection device denotes the proportionality coefficient between the detector signal and the concentration of the species, this concentration being appropriately defined.

More generally, the term "detection device" herein refers to any detection device which is useful for detecting the species (i) and (ii) at the end of the capillary tube of step (A), wherein the Taylor dispersion occurs, for example a UV detection device, an refractive index detection device, a light scattering detection device, a fluorescence detection device, or a conductivity detection device (for example of the type referred to as a "contactless conductivity detector"), a viscosity detection device, a mass spectrometer, an infrared detector, a NMR detector, an evaporative light scattering detector and the like.

Thus, the mean hydrodynamic radius of the species (i) or (ii) is a weight mean when using a mass concentration sensitive detector, a number mean when using a molar concentration sensitive detector or a z-mean when using a light scattering detector.

In fact, a great number of mixtures may be analysed according to the process of the invention, provided that they contain species which are sufficiently different in nature (as a matter of fact, a difference in nature generally implies that it exists at least one detector for which the response coefficients of the species will be distinct). Besides, a mixture analysed according to the process of the invention includes two different kinds of species, namely a monodisperse species (i) and a polydisperse species (ii).

The term "monodisperse species", as used herein, denotes a non-polymerized species (i.e. a defined molecule) or a population of polymerized macromolecules species wherein all the macromolecules have the same size.

Conversely, the term "polydisperse species" herein refers to a population of molecules, macromolecules, particles and/or aggregates which is not monodisperse, i.e. wherein the species have distinct sizes. A polydisperse species is characterized by a polydispersity index greater than 1. A polydisperse species as used according to the instant invention has preferably a polydispersity index greater than 1.05, more preferably greater than 1.1.

Especially, the method of the invention is suitable for analysing mixtures including:

- as species (i): non-polymerized molecules of a predetermined nature, and
- as species (ii): macromolecules and/or aggregates and/or particles,

for example mixtures including (i) monomers and (ii) corresponding polymers.

The method of the invention may advantageously be carried out for analysing a mixture M which is a polymerization medium, and wherein species (i) is a monomer or a mixture of monomers and species (ii) is a polymer obtained by polymerization of said species. In that specific case, at least one of the followings parameters (and preferably all of them) are determined in step (C):

- the quantity of monomer (i) in the mixture M, which indicates the degree of conversion of the polymerization; and/or
- the mean hydrodynamic radius of the polymer (ii) (that allows monitoring the size of the formed polymer. If the Mark-Houwink equation is known for the formed polymer, the weight average molar mass $M_W$ of the formed polymer may further be deduced.)

The method of the invention may besides be used for analysing other types of mixtures, such as, for example:

- mixtures including (i) proteins ; and (ii) protein aggregates and/or polymers;
- mixtures including (i) surfactants and monomers ; and (ii) polymers (which may be at least in part in the form of a latex);
- mixtures including (i) monodisperse nanoparticles ; and (ii) polymers;
- mixtures including (i) monodisperse molecules ; and (ii) nanoparticles.

The possibility of deconvolution of taylorgrams of mixtures, which has now been evidenced by the inventors, permits to directly obtain information about the mean hydrodynamic radii and the content of each of the species of the mixture, without having to preliminarily separate the constituents of said mixture. The process of the invention allows a one step very simple and fast analysis.

Moreover, the process of the invention may be carried out efficiently with extremely little quantities of the mixture to be analysed (quantities as small as 1 to 10 nL are generally injected). Furthermore, the process of the invention does not necessitate any expensive or bulky equipment.

Besides, steps (A) and (B) of the method of the invention may be implemented in almost all known capillary electrophoresis devices with no significant technical modification of these devices, which allows implementation of these steps (A) and (B) to be envisaged without any additional cost in most of existing commercial electrophoresis devices.

Especially due to these advantages, the method of the invention may be advantageously implemented both in the field of the search and on an industrial scale, especially for monitoring the evolution of reactions such as polymerization or for studying the stability of polymer / proteins formulations.

In the process of the invention, the analysis of the taylorgram (i.e. of the signal obtained in step (B)) is performed in step (C). In this step (C), the specific contributions of species (i) and (ii) on the signal obtained in step (B) are established, which allows to determine the content and/or the mean hydrodynamic radius of species (i) and/or (ii).

According to a first embodiment the first species (i) being known to be monodisperse, the response coefficient of the species (i) and (ii) being known and the detection device implemented in step (B) being a device on which the species (i) an (ii) have two distinct response coefficients, step (C) is carried out as follows:

- performing an initial Taylor Dispersion experiment on species (i) only to derive the value of the area $A_0$, the height $h_0$ and standard deviation $\sigma_i$ from an initial signal $S_0(t)$ ; and,

- on the global signal $S_n(t)$ provided at the step (B) on sample n, calculating: $\alpha_n = \dfrac{h_n}{h_0}\dfrac{A_0}{A_n}$ and $\beta_n = \dfrac{\sigma_n^2}{\sigma_i^2}$, where $A_n$, $h_n$ and $\sigma_n^2$ are respectively the area, the peak height and the variance of the global signal;

- calculating the degree of conversion of the polymerization detection response factor $k_{ii}/k_i$, through the determination of: $\Psi_n = \dfrac{y_n}{y_n + \kappa(1 - y_n)}$, where $\kappa$ is the ratio of the

$$x_n = \frac{\sigma_{ii}}{\sigma_i} = \frac{1}{2}\left(\sqrt{\frac{4\beta_n - 3 - \alpha_n}{1 - \alpha_n}} - 1\right) \text{ and } y_n = \frac{A_{ii}}{A_n} = (\beta_n - \alpha_n)\frac{x_n}{x_n^3 - 1}.$$

When two species (i) and (ii) have distinct hydrodynamic radii, they generally have distinct response coefficients on a light scattering detection device. Thus, with such species, the method according to the first embodiment of the invention may be carried out by implementing a light scattering detection device.

However, with such species having distinct hydrodynamic radii (especially monomers and corresponding polymers; or proteins or proteins aggregates), another method may be employed, which takes profit of this difference of hydrodynamic radii. In this case, the difference of hydrodynamic radii offers the possibility of directly establishing the contributions of species (i) in the signal obtained in step (B) by a deconvolution of the signal.

More precisely, according to a second embodiment which is suitable when the species (i) an (ii) have two distinct hydrodynamic radii and when species (i) is a species of a predetermined nature, the method is carried out such that, in step (C), the respective contributions of species (i) and (ii) in the signal obtained in step (B) are established, whereby the elution profiles of species (i) and (ii) are obtained, which allows to determine the content of species (i) and/or (ii) in the mixture M, and/or the mean hydrodynamic radius of the species (i) and/or (ii).

This second embodiment of the method is especially advantageous since it does not need any initial Taylor experiment.

The method according to the second embodiment is especially suitable for analysing mixtures M of the type including:

(i) non-polymerized molecules of a predetermined nature, and
(ii) macromolecules and/or aggregates and/or particles.

With such mixtures, the method according to the second embodiment of the invention allows to establish in step (C):

- the content of species (i) and/or (ii) in the mixture M ; and/or
- the mean hydrodynamic radius of species (ii).

More generally, the method according to the second embodiment of the invention is suitable for analyzing any mixture containing (i) monodisperse species of a predetermined nature (such as proteins for example); and (ii) aggregates and/or particles (proteins aggregates for example).

According to a first alternative of the method of the second embodiment considering that the elution profile of species (i) is a Gaussian distribution, the respective contributions of species (i) and (ii) in the signal obtained in step (B) are established in step (C) by:

- fitting a first Gaussian distribution onto the global signal $S_n(t)$, resulting in a first fitted Gaussian distribution corresponding to the elution profiles of species (i);
- subtracting from the signal the first fitted Gaussian distribution, resulting in a reduced signal $S'_n(t)$ corresponding to the elution profile of species (ii) and providing information about species (ii).

In this first alternative, preferably, the reduced signal $S'_n(t)$ is processed to obtain a value of the variance for species (ii) according to:

$$\sigma_{ii,n}^2 = \frac{\int_{t_{d,n}-b}^{t_{d,n}+b} S'_n(t).(t-t_{d,n})^2.dt}{\int_{t_{d,n}-b}^{t_{d,n}+b} S'_n(t).dt} \text{ or } \sigma_{ii,n}^2 = \frac{\int_{t_{d,n}-b}^{t_{d,n}} S'_n(t).(t-t_{d,n})^2.dt}{\int_{t_{d,n}-b}^{t_{d,n}} S'_n(t).dt}$$

where b is a period of time large enough for the signal to vanish at $t_{d,n} - b$ and at $t_{d,n} + b$.

In a second possible alternative of the second embodiment considering that the elution profile of species (i) and species (ii) are Gaussian distributions, the respective contributions of species (i) and (ii) in the signal obtained in step (B) are established, in step (C), by fitting a function which is the sum of first and second Gaussian distributions onto the signal $S_n(t)$, resulting in first and second fitted Gaussian distributions corresponding to the elution profiles of species (i) and (ii).

In the invention, mixture M is continuously injected so as to make a unit step injection. Then the respective contributions of species (i) and (ii) in the signal $T_n(t)$ obtained in step (B) are established, in step (C), by fitting onto the signal $T_n(t)$ the following fit function:

$$C_n(t) = \frac{C_0^i}{2}\left[1 - erf\left(\frac{t-t_{d,n}}{\sigma_{i,n}\sqrt{2}}\right)\right] + \frac{C_0^{ii}}{2}\left[1 - erf\left(\frac{t-t_{d,n}}{\sigma_{ii,n}\sqrt{2}}\right)\right]$$

resulting in the determination of the following parameters: $t_{d,n}$ is the time when an inflexion point is detected in the edge of the signal; $\sigma_{i,n}$ and $\sigma_{ii,n}$, the variances for the contribution of the two species (i) and (ii); and $C_0^i$ and $C_0^{ii}$, the maximal absorbances of the two species (i) and (ii).

Then, the value of the variance of the elution profile of species (ii) is used to determine, a hydrodynamic radius according to the equation:

$$R_{h,ii,n} = \frac{2kT}{3\pi\eta u}\frac{1}{H_{ii,n} \pm \sqrt{H_{ii,n}^2 - \frac{d_c^2}{12}}} \text{ with: } H_{ii,n} = \frac{l_s\sigma_{ii,n}^2}{t_d^2}$$

Preferably, the value of the area of the elution profiles of species (i) and (ii) are used to determine, the degree

$$\Psi_n = \frac{A_{i,0} - A_{i,n}}{A_{i,0}},$$

of conversion $\Psi_n$ according to the equation:   where $A_{i,0}$ and $A_{i,n}$ are the areas of the signal of species (i) in an initial experiment (time 0), for example of a monomer before the start of a polymerization, and in experiment n, for example during the polymerization process, respectively.

The mixture M being a polymerization medium, and the species (i) being a monomer and the species (ii) being polymers obtained by polymerization of said species (i), the followings are determined in step (C) :

- the quantity of monomer (i) in the mixture M, which indicates the degree of conversion of the polymerization; and/or
- the mean hydrodynamic diameter of the polymer (ii).

Preferably, the mixture M is diluted or dissolved in a medium identical to the carrier liquid used in the Taylor dispersion of step (A), before injecting it in the capillary.

The internal surface of the capillary tube is non-covalently or covalently coated with a compound which limits or inhibits the interaction between the species (i) and (ii) and the inner surface of the capillary tube, said coated compound being chosen from the group consisting in PEO, cellulose derivatives, polyvinyl alcohol, polyacrylamides, polysiloxanes such as polydimethylsiloxane, anionic or cationic (mono- or double-chain) surfactants, polyelectrolyte mono or multilayer(s).

The mixture M is introduced in the capillary tube together with a carrier liquid (a good solvent of the solute mixture). When using water or hydro-organic solvents, the carrier liquid generally includes a salt, for example NaCl, or a buffered solution.

The method, wherein a plurality of samples, which each include a mixture of at least two species and having two different response coefficients on at least one detection device, are analysed according to steps (A) to (C) as defined here above in the same capillary tube, in a sequential way, the samples being injected successively in said capillary tube.

The object of the invention is also an apparatus for a Taylor experiment, comprises a detector and an analysis device, said detector comprises a capillary tube through which flows the mixture M to be analysed, injection means (6) for the injection of the mixture M into the capillary tube, and at least one set of optical means to produce a Taylor signal, and said analysis device comprising memorisation means, processing means, an I/O interface to receive from said detector said Taylor signal, and means for the implementation of the method of the invention.

Different variants and preferred embodiments of the method of the invention will now be described in greater details with reference to the attached drawings, on which:

- Figure 1 is a schematical block illustration of an apparatus for Taylor experiments;
- Figure 2 is an algorithmic representation of the method of treatment performed by the apparatus of figure 1;
- Figure 3 represents Taylorgrams obtained for different reaction times for a mixture according to Example 1;
- Figure 4 represents the degree of conversion of the reaction determined from the Taylorgrams of Figure 3 by a first method according to the invention;
- Figure 5 shows the evolution of the hydrodynamic radius with the reaction time, as determined by the three first methods according to the invention, which involve plug injection of the samples to be analysed; and,
- Figure 6 represents the superposition of the signals obtained by Taylor dispersion analysis of a polymer / monomer mixture and of the polymer alone for a fourth method according to the invention, which involve step injection of the samples to be analysed.

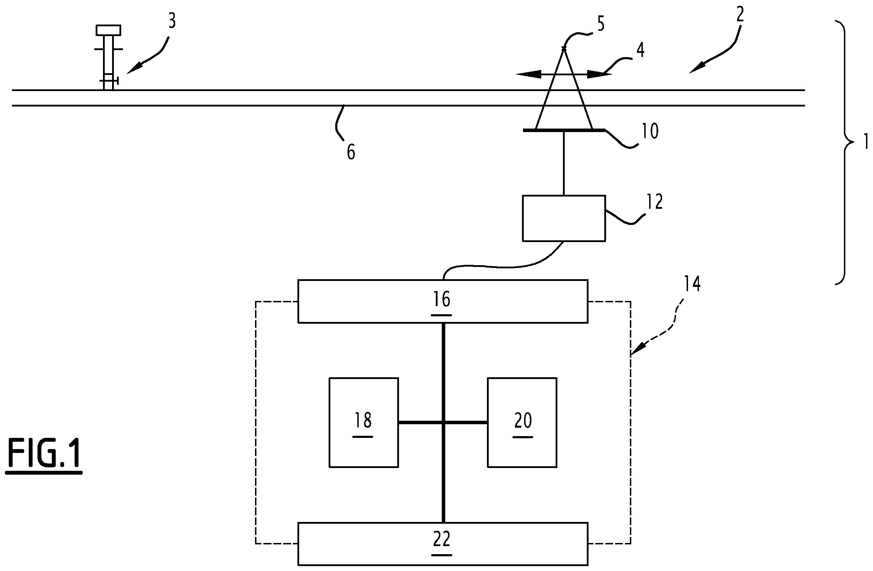

As illustrated in figure 1, an apparatus 1 for a Taylor experiment. Apparatus 1 comprises a detector 2 and an analysis device 14.

Detector 2 contains a capillary tube 6 through which flows the mixture to be analysed. At the input end of the capillary tube, detector 2 contains injection means 3 for the injection of the mixture to be analysed. Preferably, the injection means 3 allows a unit pulse injection (i.e. a plug injection) or a unit step injection (i.e. a continuous injection of the sample) to be performed. The injection means allows a constant pressure injection or, in another embodiment, a constant flow injection.

Near the other end of the capillary tube, the detector 2 contains a light source S and an optical system 4 to irradiate the capillary tube 6. On the other side of the tube, along the optical axle of the optical system 4, the detector 2 contains an optical sensor 10 coupled to an electronic board 12 capable of generating an electrical signal S. The level of signal S depends directly on the amount of light incident on sensor 10 during a period of time equal to the sampling time.

Detector 2 is connected to an analysis device 14 through an Input/Output interface 16. Device 14 further comprises memorisation means, such as RAM and/or ROM 18, and processing means, such that CPU 20. Device 14 is also provided with means to allow an operator to interact with software running on device 14. For example, device 14 is equiped with a tactile screen 22.

The method of treatment of the electrical signal S that will be described hereafter in details is preferably performed by a software comprising instructions memorised in ROM 18 and processed by CPU 20.

In step (A), the mixture M, as such or dissolved and/or dispersed in a proper solvent or dispersant, is injected in the capillary tube and forced to be transported in this capillary tube by the flow of a carrier liquid induced by a positive hydrodynamic and/or hydrostatic pressure difference between the inlet and the outlet of the capillary.

The capillary tube 6 used in step (A) has advantageously an inner diameter of between 5 and 500 $\mu$m. The inner diameter of the capillary is advantageously less than or equal to 300 $\mu$m, preferably less than or equal to 100 $\mu$m, especially in order to prevent an excessively high level of dispersion of the peaks. However, it is preferable for this inner diameter to remain greater than or equal to 10 $\mu$m, in particular in order to allow adequate sensitivity of measurement and also to provide for conditions under which the plate height H is a linear function of the mobilizing velocity u. Thus, typically, the inner diameter of the capillary tube used in step (A) is between 25 and 100 $\mu$m. Thus, as capillaries which can advantageously be used to implement step (A), mention may be made to conventional capillaries having an inner diameter of 10 $\mu$m, 25 $\mu$m, 50 $\mu$m, 75 $\mu$m or 100 $\mu$m, capillaries of 25, 50 and 75 $\mu$m being found to be particularly suitable in most cases.

As it is well known in the field of the Taylor dispersion, the length l of the capillary tube implemented in the method of the invention has to be sufficiently high so as to obtain a detection time well greater than the characteristic diffusion time (which is calculated by the ratio $R^2/4D$ wherein R is the internal radius of the capillary tube 6 and D is the diffusion coefficient). To this end, the length of the capillary tube used in the method of the invention is advantageously of at least 10 cm, preferably of at least 20 cm. Besides, especially in order to limit the analysis times, it is preferable for the length of the capillary to remain less than 100 cm, for example, less than or equal to 50 cm. In the meaning of the instant description, the term "length of a capillary tube" intends to denote the effective length of the tube, namely the length from the inlet to the optical sensor 10 located in the region of the outlet of the capillary tube.

The Taylor dispersion which is conducted in step (A) may be carried out in accordance with any method known per se, for example, in accordance with the technique described in J. Phys. Chem., 1974, 78, 2297-2301 or in Science, 1994, 266, 773-776.

In the method of the invention, the Taylor dispersion is brought about by establishing in step (A) a positive hydrodynamic and/or hydrostatic pressure difference between the inlet and the outlet of the capillary tube.

In step (A) of the method of the invention, it is highly preferable that the linear displacement velocity u of the

$$7\frac{D}{R} < u << \frac{4Dl}{R^2}$$

species (i) and (ii) in the capillary tube fulfils the following relation:

It is well known by the skilled person to adapt the hydrodynamic and/or hydrostatic pressure difference induced between the inlet and the outlet of the capillary tube so as to attain the sought rate of flow for a given capillary. For example, in the case of species having diffusion coefficients of about $10^{-9}$ to $10^{-11}$ m$^2$s$^{-1}$, and for a capillary tube having an internal diameter of 50 $\mu$m and an efficient length of 30 cm, the mobilisation rate has to be between $5.10^{-4}$ and $2.10^{-3}$ ms$^{-1}$, which corresponds to a pressure difference between the inlet and the outlet of the capillary tube of about 10 to 50 mbar ($10^3$ to $5.10^3$ Pa). For species having diffusion coefficients of less than $10^{-11}$ m$^2$s$^{-1}$, it often reveals suitable to make use of capillary tube having an internal diameter of less than 50 $\mu$m so as to obtain reasonable mobilisation rates and analysis times. More generally, the hydrodynamic and/or hydrostatic pressure difference between the inlet and the outlet of the capillary in step (A) may be from 1 mbar to 1 bar ($10^2$ Pa to $10^5$ Pa) especially for capillary tube having an internal diameter of 25 to 100 $\mu$m. Greater pressure difference up to 6 bars will be more suitable for capillary tube having an internal diameter of less than 25 $\mu$m and/or for longer capillary tubes (10 or 20 m for instance).

Furthermore, it is generally preferable for the pressure difference applied during step (A) between the ends of the capillary to remain substantially constant for the entire duration of step (A), in particular in order to allow the most precise measurement possible of the hydrodynamic radius in step (C). In this manner, advantageously, during step (C), the pressure varies at the most within +/- 0.5 mbar (50 Pa) of a fixed reference value. However, the value of this reference value most generally does not have to be determined in a precise manner. Other possibility is to perform the Taylor experiment by setting and controlling the flow rate instead of the pressure.

In step (A), the pressure difference between the two ends of the capillary may be established in accordance with any method known per se, for example, by applying excess pressure in the region of the inlet of the capillary or, conversely, by applying reduced pressure at the outlet. According to a more specific embodiment, the pressure difference between the two ends of the capillary may be brought about by establishing a level difference between reservoirs of solvent or mobile phase at the inlet and at the outlet of the capillary. This embodiment is generally found to be advantageous since it allows a constant pressure difference to be established for the entire duration of step (A) without requiring any additional pressure regulation system.

In step (A), it is generally preferable to dilute or dissolve the mixture M in a medium identical to the carrier liquid used in the Taylor experiment before injecting it in the capillary tube 6. This pre-dilution or pre-solubilisation of the mixture M especially allows:

-    a limitation or an inhibition of baseline perturbations of the signal obtained in step (B) which may otherwise occur (especially when the detection device located in the region of the outlet of the capillary tube is a UV-detector: in the absence of a dilution or solubilisation of the mixture, modifications of the refraction index of the mixture may occur in the injection zone). This "stabilisation" of the signal obtained in step (B) renders more efficient the analysis of step (C).

-    a limitation or an inhibition of modification of the conformation of macromolecules (polymers or proteins for example) present in the mixtures during the Taylor dispersion experiment. The dilution or solubilisation tends to level off the change in ionic strength between the mixture zone and the carrier liquid.

-    a lower change in the viscosity between the mixture zone and the mobile phase.

-    a limitation of intermolecular interactions between sample (macro)molecules that are assumed to be negligible for calculation of the radius $R_h$.

Besides, in step (A), the internal surface of the capillary is preferably non-covalently or covalently coated with a compound which limits or inhibits the interaction (especially adsorption) between the species (i) and (ii) and the inner surface of the capillary, said coated compound being preferably chosen from the group consisting in PEO, cellulose derivatives, polyvinyl alcohol, polyacrylamides, polysiloxanes such as polydimethylsiloxane, or surfactants (mono-chain or double chain surfactants). Polyelectrolyte multilayer coatings may be applied on the internal face of the capillary tube. Commercial coatings such as DB1, DB17 or DB225 are especially useful in this connection.

The inhibition of the interactions between the capillary and the species (i) and (ii) improves the quality of the signal obtained in step (B) and hence facilitates the analysis of this signal in step (C).

Any other means allowing to limit or inhibit interactions between species (i) and (ii) may be advantageously implemented in the step (A). Especially, according to a specific embodiment, it may reveals advantageous that the mixture M is introduced in the capillary tube together with a carrier liquid including a salt, for example NaCl.

According to an advantageous alternative, the method of the invention may be conducted so as to sequentially analyse a plurality of N samples. In this alternative, a plurality of samples, which each includes a mixture of at least two species and having two different response coefficients on at least one detection device, are analysed, according to steps (A) to (C) as defined in claim 1, in the same capillary tube, in a continuous way, the samples being injected successively in said capillary tube. According to this embodiment, it is not necessary to wait the detection of a first sample detected and analysed according to step (B) and (C) before introducing the following sample. Thus, in this connection, the instant invention provides an efficient and fast analysis method, which allows a high flow of sample. This embodiment of the method of the invention is, inter alia, especially suitable for a continuous study of the evolution of a polymerization.

By using device 14 and detector 2 on the $n^{th}$ sample of mixture M of monomer m and polymer p, the signal $S_n(t)$ given by the sensor at the end of step (B) is the superposition of the signal $S_{m,n}(t)$ of the monomer m and the signal $S_{p,n}(t)$ of the polymer p.

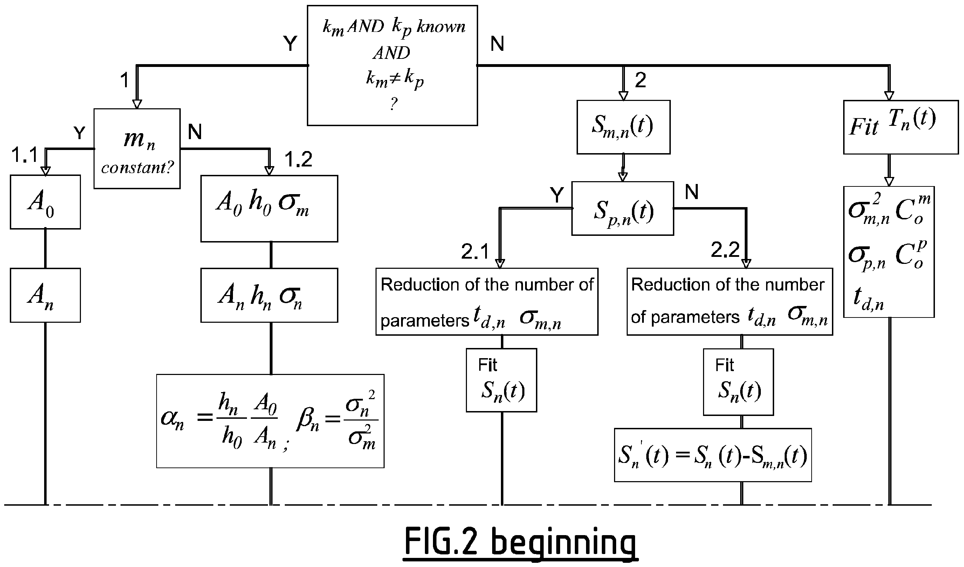

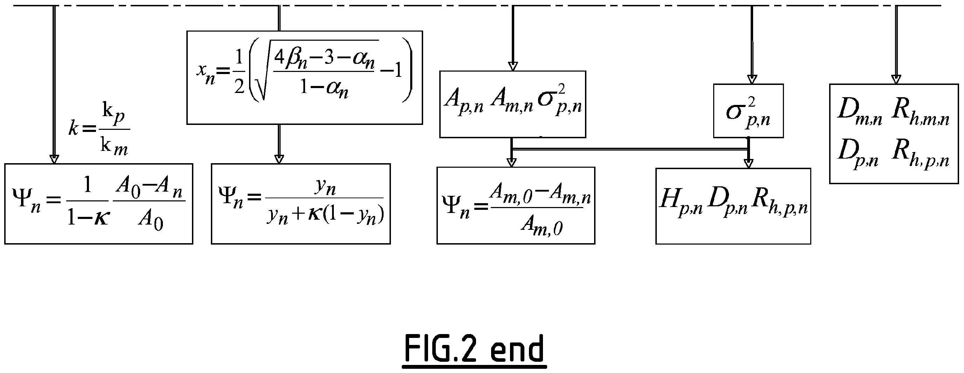

To derive further physical data on these two species, such that the degree of conversion of the polymerisation reaction and/or the mean hydrodynamic radius of the polymer p, each contribution to the global signal $S_n(t)$ must be separated by applying one of the following methods of treatment of the global signal $S_n(t)$, as it can be seen in figure 2.

For example, the degree of conversion $\Psi_n$ of the polymerisation reaction at the time sample n is taken off, is defined as:

$$\Psi_n = \frac{m_{p,n}}{m_{m,n} + m_{p,n}} \tag{1}$$

where $m_{m,n}$ and $m_{p,n}$ are the masses, respectively of the monomer m and the polymer p, in sample n.

## 1. The response coefficients of each species are known

The response coefficient k for one species is defined by:

$$A_{m,n} = k_m . m_{m,n} \tag{2}$$

$$A_{p,n} = k_p . m_{p,n} \tag{3}$$

where $A_{m,n}$ and $A_{p,n}$ are the areas of the corresponding signals $S_{m,n}(t)$ and $S_{p,n}(t)$.

Coefficients $k_m$ and $k_p$ are considered constant throughout the experiment, even if the structure of a species, like the one of the polymer p, can evolve.

## 1.1. With a condition of conservation of the mass

The mass of sample n can be expresses as the sum of the mass of the monomer and the mass of the polymer:

$$m_n = m_{m,n} + m_{p,n} \tag{4}$$

where $m_n$ can be considered as the mass introduced in the device for experiment n.

One hypothesis is to consider $m_n$ constant, and equal to $m$, from one sample to the other.

The total area $A_n$ of total signal $S_n(t)$ is:

$$A_n = k_m m_{m,n} + k_p m_{p,n} \tag{5}$$

At the beginning (n=0) of the polymerisation, the sample only contains a mass $m$ of monomer, so:

$$A_0 = k_m.m_{m,0} = k_m.m \tag{6}$$

Now, equation (1) can be written as:

$$\Psi_n = \frac{1}{1-\kappa}\frac{A_0 - A_n}{A_0} \tag{7}$$

with:

$$\kappa = \frac{k_p}{k_m} \tag{8}$$

Thus, the degree of conversion $\Psi_n$ can be directly calculated from the total area $A_0$ and $A_n$ of the signal before polymerisation (n=0) and at the current time n.

This is possible when the mass of the sample is constant and if $k_p$ is different from $k_m$ in order the denominator of equation (7) does not vanish.

### 1.2. Without a condition of conservation of the mass

When considering that the condition about the mass of the sample which must be constant throughout several samples is too restrictive or difficult to put into practice, the following method can be used.

Assuming that the mean time $t_{d,m,n}$ and $t_{d,p,n}$ of the two signals $S_{m,n}(t)$ and $S_{p,n}(t)$ are the same, and that the two signals $S_{m,n}(t)$ and $S_{p,n}(t)$ are symmetrical around this common mean time $t_{d,n}$, then the maximum $h_n$ of the global signal $S_n(t)$ arises at $t_{d,n}$ and is given by:

$$h_n = h_{m,n} + h_{p,n} \tag{9}$$

where $h_{m,n}$ and $h_{p,n}$ are the heights of the peaks of the signals $S_{m,n}(t)$ and $S_{p,n}(t)$, respectively.

Similarly, the variance $\sigma_n^2$ of the global signal $S_n(t)$ can be expressed, by:

$$\sigma_n^2 = \frac{A_{p,n}}{A_{m,n} + A_{p,n}}\sigma_{p,n}^2 + \frac{A_{m,n}}{A_{m,n} + A_{p,n}}\sigma_{m,n}^2 \tag{10}$$

where $\sigma_{m,n}^2$ and $\sigma_{p,n}^2$ are the variances of the $S_{m,n}(t)$ and $S_{p,n}(t)$ signals, respectively.

By introducing parameter $y_n$ defined by:

$$y_n = \frac{A_{p,n}}{A_{m,n} + A_{p,n}} \tag{11}$$

it is easy to derive that:

$$y_n = \frac{\kappa \, \Psi_n}{1 - \Psi_n + \kappa \Psi_n} \tag{12}$$

or, that:

$$\Psi_n = \frac{y_n}{y_n + \kappa(1 - y_n)} \tag{13}$$

The two parameters of interest are $\sigma_p^2$ and $\Psi_n$. The latter is connected to the areas $A_{m,n}$ and $A_{p,n}$ via equations (11) and (13).

The method involves the introduction of two new variables, the values of which are accessible from the experiment:

$$\alpha_n = \frac{h_n}{h_0} \frac{A_0}{A_n} \tag{14}$$

$$\beta_n = \frac{\sigma_n^2}{\sigma_m^2} \tag{15}$$

It can be shown that:

$$\alpha_n = 1 - y_n + \frac{y_n}{x} \tag{16}$$

$$\beta_n = 1 - y_n + y_n x^2 \tag{17}$$

where parameter $x_n$ is defined by:

$$x_n = \frac{\sigma_{p,n}}{\sigma_{m,n}} \tag{18},$$

Equations (16) and (17) lead to:

$$x_n = \frac{1}{2}\left( \sqrt{\frac{4\beta_n - 3 - \alpha_n}{1 - \alpha_n}} - 1 \right) \tag{19}$$

$$y_n = (\beta_n - \alpha_n)\frac{x_n}{x_n^3 - 1} \tag{20}$$

and, the value $\Psi_n$ can be derived from $y_n$ using equation (13) and $\sigma_p$ using equation (18).

Thus, according to this method, the measurements of the area $A_0$, the height $h_0$ and the standard deviation $\sigma_m$ of the signal $S_{m,0}(t)$ of the monomer before the polymerisation reaction, allow the determination of the degree of conversion

$\Psi_n$ and of the standard deviation $\sigma_p$ of the polymer.

An illustration of method 1 above is described in the following example.

**Example 1**: Analysis of the Taylor dispersion of a polymerisation reaction (polyacrylamide)

In this example, a polymerisation reaction has been carried out and the evolution of the composition of the reaction medium has been determined by making use of method 1 as described above.

The polymerisation reaction is a radical polymerization of a acrylamide in presence of the redox couple persulfate/N,N,N',N'-tertraméthyl-ethylene-diamine as a radical initiator. The polymerisation has been carried out in a water/ethanol mixture (80/20 w/w) under a $N_2$ atmosphere at 25°C, with a initial content of acrylamide monomer in the reaction medium of 0.1 M.

The degree of conversion of the monomer has been studied by taking samples of the reaction medium at different reaction times. For each samples, 10 microliters of the reaction medium have been taken and immediately diluted 100 times in 1 mL of a aqueous solution of NaCL (0,1 M). The resulting diluted sample has been introduced in liquid nitrogen so as to stop the polymerization reaction.

Each of the sample have been next analyzed according to the method of the invention by making use of a capillary electrophoresis device (Agilent Technologies) making use of a capillary DB1 (coated capillary commercialized by JW Scientific) having a length of 31 cm (25 cm from the inlet to the detector) and an inner diameter of 50 $\mu$m. The samples have been introduced hydrodynamically, by application of a pressure of 17 mbar during 10 s (which corresponds to an injected volume of about 10 nl). The mobilization of the solute has been made at a constant pressure of 30 mbar. The mobile phase used is an aqueous solution of NaCl (0.1 M). The presence of salt limits the interaction between the polymer and the walls of the capillary. Furthermore, it fixes the ionic force.

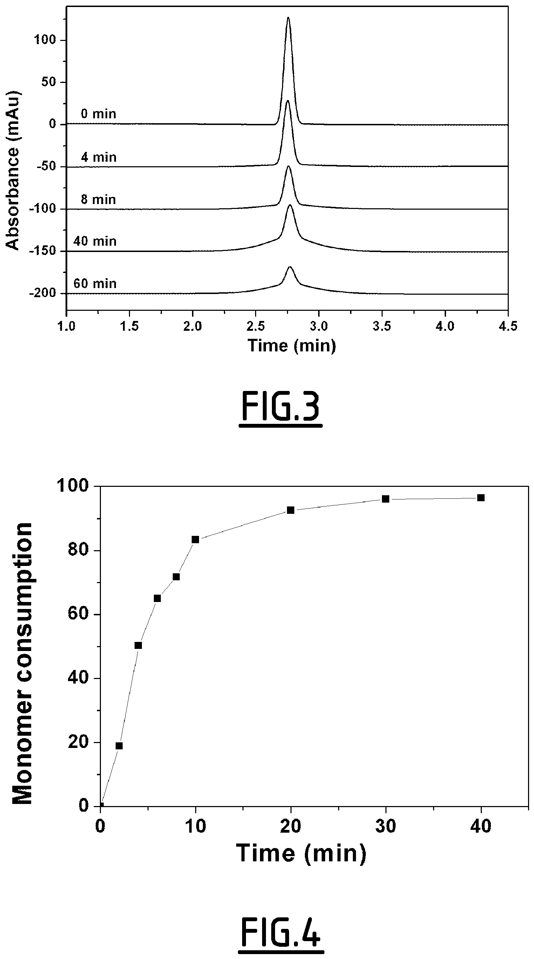

The Taylorgram obtained for different reaction times are reported on attached Figure 3. According to the method of the invention, the evolution of the degree of conversion of the reaction is calculated and shown on the attached Figure 4.

## 2. Hypothesis on the response signals

Considering that the signal $S_{m,n}(t)$ of the monomer is a Gaussian distribution, we have:

$$S_{m,n}(t) = \frac{A_{m,n}}{\sigma_{m,n}\sqrt{2.\pi}} . \exp\left\{-\frac{1}{2}\left[\frac{t-t_{d,m}}{\sigma_m}\right]^2\right\} \qquad (21)$$

where $\sigma_m$ is the standard deviation, $t_{d,m}$ the mean value and $A_{m,n}$ a normalisation factor corresponding to the area of the signal $S_{m,n}(t)$.

### 2.1.

The signal of the polymer $S_{p,n}(t)$ can also be approximated with a Gaussian distribution:

$$S_{p,n}(t) = \frac{A_{p,n}}{\sigma_{p,n}\sqrt{2.\pi}} . \exp\left\{-\frac{1}{2}\left[\frac{t-t_{d,p}}{\sigma_{p,n}}\right]^2\right\} \qquad (22)$$

This is the case when the dispersion in molar mass of the polymer p is not too large.

Thus, a first method to separate each contribution consists in fitting a function which is the sum of two Gaussian distributions onto the global signal $S_n(t)$.

This fit involves six parameters, namely $\sigma_m$, $\sigma_{p,n}$, $t_{d,m}$, $t_{d,p}$, $A_{m,n}$ and $A_{p,n}$.

It is possible to reduce the number of parameters in order to get a better precision from the fit.

For example, $\sigma_m$ is a characteristic parameter of the monomer. It can be measured trough an initial Taylor experiment with a solution containing only the monomer.

It is to be noticed that because Taylor dispersion is not a method of separation of the compounds of the mixture, $t_{d,m}$ is egal to $t_{d,p}$, and is also equal to the mean value $t_{d,n}$ of the global signal $S_n(t)$.

**2.2.**

When the molar mass distribution of the polymer p is too polydispersed, it is no longer possible to fit a Gaussian distribution onto the global signal $S_n(t)$. In this case, the method consists in first fitting a Gaussian distribution onto the signal of the monomer $S_m(t)$, then subtracting the fitted Gaussian distribution from the signal $S_n(t)$ to obtain a reduced signal $S'_n(t)$ and then calculating the variance of the reduced signal $S'_n(t)$ as an approximation of $\sigma_{p,n}^2$ :

$$\sigma_{p,n}^2 = \frac{\int_{t_{d,n}-b}^{t_{d,n}+b} S'_n(t).(t-t_{d,n})^2.dt}{\int_{t_{d,n}-b}^{t_{d,n}+b} S'_n(t).dt} \qquad (23)$$

This method works well when the characteristic parameters of the signal of the monomer $S_{m,n}(t)$ are significantly different from those of the signal of the polymer $S_{p,n}(t)$, i.e. when the signal of the monomer $S_{m,n}(t)$ can be "visually" separated from the signal of the polymer $S_{p,n}(t)$. For example, when the signal of the monomer $S_{m,n}(t)$ is narrow and sharp whereas the signal of the polymer $S_{p,n}(t)$ is broad (i.e. when the polymer has much larger molar mases than the monomer).

The implementation of this method in device 14 can be done by fitting a Gaussian distribution onto the global signal $S_n(t)$ but only inside a reduced window centered on the maximum height of the global signal.

Alternatively, an iteration process can be used: in a loop of this iteration process, a fit corresponding to the monomer signal is adjusted on the global signal $S_n(t)$. The fitted Gaussian distribution is subtracted from the global signal $S_n(t)$ to get the reduced signal $S'_n(t)$. Then, the shape of the reduced signal $S'_n(t)$ is tested. This test may consist in calculating the number of zeros of the derivative of the reduced signal $S'_n(t)$ : if the derivative has three zeros, it means that the area of the fitted Gaussian distribution is too large and that a part of the polymer signal has been considered as belonging to the monomer signal. Thus, in the following loop of the iteration process, the area of the Gaussian to be fitted is decreased. On the contrary, if the derivative has only one zero, it means that the area of the fitted Gaussian distribution is too small and that a part of the monomer signal has not been taken into account in the monomer signal. Thus, in the following loop of the iteration process, the area of the Gaussian to be fitted is increased. The convergence of such an iteration process results in a limit by excess of the area of the monomer signal. Another criterion can be used such that the number of zeros of the second derivative of the resulted signal $S'_n(t)$.

In experimental conditions, it may happen that the signal $S_{p,n}(t)$ of the polymer has a tail due to an absorption phenomenon of the polymer on the wall of the capillary tube. In order to avoid the calculation of $\sigma_{p,n}^2$ be biased by this phenomenon and considering the signal as symmetrical, equation (23) becomes:

$$\sigma_{p,n}^2 = \frac{\int_{t_{d,n}-b}^{t_{d,n}} S'_n(t).(t-t_{d,n})^2.dt}{\int_{t_{d,n}-b}^{t_{d,n}} S'_n(t).dt} \qquad (24)$$

It is to be noticed that $A_{m,n}$ can be derived from the result of the fitting of a Gaussian distribution and that $A_{p,n}$ can be obtained by subtracting $A_{m,n}$ from $A_n$. From the deconvolution of the respective contributions of the monomer and the polymer, it is possible to get the degree of conversion $\Psi_n$ for sample n:

$$\Psi_n = \frac{A_{m,0}-A_{m,n}}{A_{m,0}} \qquad (25)$$

These data on the elution profiles of the monomer and the polymer can be used to determine physical characteristics such that the hydrodynamic radius of the polymer. The radius $R_{h,p,n}$ is connected to the diffusion coefficient $D_{p,n}$ by means of the Stokes-Einstein relationship:

$$R_{h,p,n} = \frac{kT}{6\pi\eta D_{p,n}} \qquad (26)$$

and the diffusion coefficient $D_{p,n}$ is connected to the variance $\sigma^2_{p,n}$ through parameter $H_{p,n}$ :

$$D_{p,n} = \frac{u}{4}\left(H_{p,n} \pm \sqrt{H^2_{p,n} - \frac{d_c^2}{12}}\right) \qquad (27)$$

$$H_{p,n} = \frac{l_s . \sigma^2_{p,n}}{t_d^2} \qquad (28)$$

where $l_s$ is the length of the capillary tube from the injection point to the detector and $u$ is the flow velocity of the carrier solvent through the tube, $\eta$ is the viscosity of carrier liquid at temperature T. So, once $\sigma^2_{p,n}$ has been measured for sample n, a hydrodynamic radius $R_{h,p,n}$ can be derived for the polymer of this nth sample.

In the software implementation of this method, $D_{p,n}$ is only an intermediary parameter. It is not necessary to use it explicitly in the calculation of the value of the radius $R_{h,p,n}$ which can be derived directly from the input of $\sigma^2_{p,n}$ :

$$R_{h,p,n} = \frac{2kT}{3\pi\eta u}\frac{1}{H_{p,n} \pm \sqrt{H^2_{p,n} - \frac{d_c^2}{12}}} \quad \text{with} \quad H_{p,n} = \frac{l_s \sigma^2_{p,n}}{t_d^2} \qquad (29)$$

For seek of comparison, the results obtained according to the method 1, 2.1 and 2.2 described above have been compared as regards the polymerisation described in Example 1.

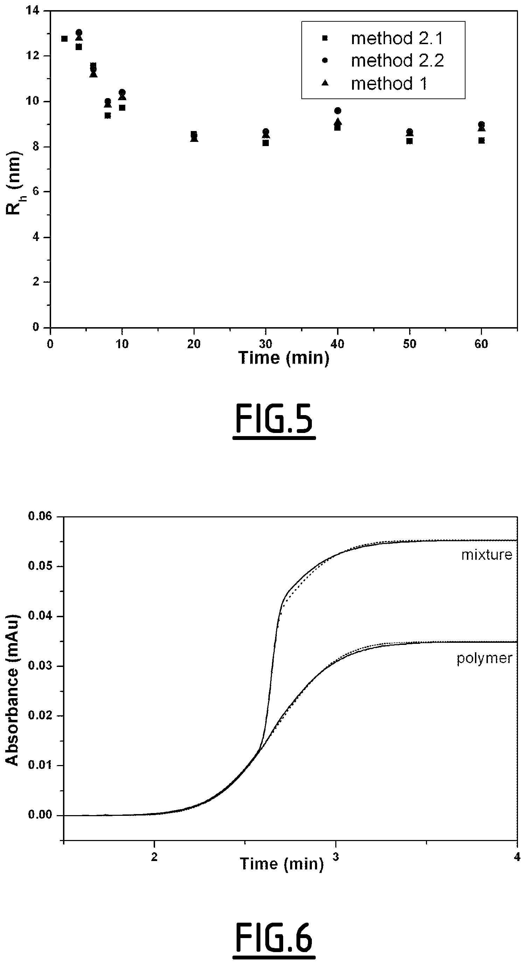

Attached Figure 5 shows the evolution of hydrodynamic radius with the reaction time, as determined by the three methods.

### 3. Taylor analysis with a step injection

Another way to use the previous described apparatus 1 is to inject the mixture to be analysed so as to make a unit step function (continuous sample injection) at the inlet end of the capillary tube, rather than a unit pulse function (plug injection) as it was generally the case in the here above methods. The step injection is performed under the constraint of a constant flow rate, instead of setting a constant pressure.

Due to Taylor dispersion, the edge of the unit step function is smoothed when travelling towards the output end of the capillary tube where is positioned the optical sensor. The sensor outputs an electrical signal $T_n(t)$.

The principle of the signal analysis is here to fit, on the signal $T_n(t)$, the following fit function, which is the sum of two deformed step functions.

$$C_n(t) = \frac{C_0^i}{2}\left[1 - erf\left(\frac{t - t_{d,n}}{\sigma_{i,n}\sqrt{2}}\right)\right] + \frac{C_0^{ii}}{2}\left[1 - erf\left(\frac{t - t_{d,n}}{\sigma_{ii,n}\sqrt{2}}\right)\right] \qquad (30)$$

The fit procedure leads to the determination of five parameters, namely:

- $t_{d,n}$ the time when an inflexion point is detected in the edge of the signal;
- $\sigma_{i,n}$ and $\sigma_{ii,n}$, the variances for the contribution of the two species (i) and (ii);

- $C_0^i$ and $C_0^{ii}$, the maximal absorbance of the two species (i) and (ii).

Hereafter is described a particular experiment involving Taylor analysis of a mixture injected so as to make a unit step function. The mixture is made of monomer (acrylamide, species (i)) and a standard of polyacrylamide ($M_w$=350×10³ g/mol, species (ii)) in aqueous solution with 0.1 M NaCl. The concentrations of acrylamide and polyacrylamide in the mixture are respectively of 0.07 g/L and 0.63 g/L. Acrylamide is a small molecule in comparison to the polymer. The mixture is injected continuously into a capillary tube filed with a 0.1 M NaCl aqueous solution. Figure 6 represents the superposition of the signals $T(t)$ obtained by Taylor dispersion analysis for the polymer / monomer mixture and for the polymer alone.

The results of the fit procedure on the signal of the mixture are, for the acrylamide, $t_d$ = 2.52 min, $\sigma_{1,n}$ = 0.0315 min, $C_0^I$ = 23.3 mUA, and for the polyacrylamide, $t_d$ = 2.52 min, $\sigma_{2,n}$ = 0.269 min, $C_0^2$ = 32 mUA.

The diffusion coefficients $D$ for each species (i) and (ii) are calculated according to the previous described method with unit pulse injection. The diffusion coefficient $D$ of a species is given by equation (27):

$$D_{p,n} = \frac{u}{4}\left(H \pm \sqrt{H^2 - \frac{d_c^2}{12}}\right) \tag{27}$$

where parameter $H = \frac{l_s . \sigma^2}{t_d^2}$ is connected to the variance $\sigma^2$ of the considered species. As a reminder, $l_s$ is the length of the capillary tube 6 from the injection point to the sensor, $u = \frac{l_s}{t_d}$ is the velocity of the carrier liquid through the tube (m.s⁻¹), $\eta$ is the viscosity of carrier liquid at temperature T. From $D$, the radius $R_h$ can be derived by means of the Stokes-Einstein relationship:

$$R_h = \frac{kT}{6\pi\eta D} \tag{31}$$

The results are, for acrylamide, $D_i$ = 1.17.10⁻⁹ m².s⁻¹ and $R_{h,ii}$ = 0.21 nm, and, for the polyacrylamide, $D_{ii}$ = 1.51.10⁻¹¹ m².s⁻¹ and $R_{h,i}$ = 16.2 nm. For the calculation of the radius $R_h$, the value of the viscosity of the 0.1M NaCl solution is $\eta$= 0.89×10⁻³ Pa.s. It is worth noting that the average hydrodynamic radius of the polymer obtained from the Taylor analysis of the mixture is in very good accordance with the value obtained from the Taylor analysis of solution containing the polymer alone (without acrylamide): $R_h$=16.1 nm.

It is to be noticed that the condition of validity of the method is respected. Indeed, the period of time the mixture and the solvent stay in the capillary tube is above a characteristic diffusion time on a section of the capillary tube:

$$t_d \gg \frac{R_c^2}{4D}$$

The numerical application leads to, $\frac{R_c^2}{4D}$ = 0.13 for acrylamide, and $\frac{R_c^2}{4D}$ =10.3 s for polyacrylamide. These characteristic diffusion times must be compared with the 151 s of the detection time.

## 4. About multiple detection

It is particularly advantageous to perform a Taylor dispersion analysis with an apparatus having two optical sensors located in two different detection points A and B. Double detection avoids the corrections on detection time and on the variance due to finite length of the injection plug and due to the pressure ramp.

Thus, for a given species (i), the difference of the variances obtained at the two detection points A and B is directly linked to the diffusion coefficient according to the following equation:

$$\sigma_{i,B}^2 - \sigma_{i,A}^2 = \frac{d_c^2\left(t_{d,B} - t_{d,A}\right)}{96D_i} \qquad (32)$$

Then for analysing a mixture, it is possible to apply one of the methods previously described on a couple of electric signals, each signal being obtained in a different detection points A and B. For example, for a mixture of monomer and polymer, two sets of three parameters are used to fit the two signals:

- $\sigma_{A,p,n}^2$ : variance of species (ii), for example the polymer, at the first detection point;
- $\sigma_{A,m,n}^2$ : variance of species (i), for example the monomer, at the first detection point;
- $t_{d,A}$ : the mean detection time at the first detection point;
- $\sigma_{B,p,n}^2$ : variance of species (ii), for example the polymer, at the second detection point;
- $\sigma_{B,m,n}^2$ : variance of species (i), for example the monomer, at the second detection point;
- $t_{d,B}$ : the mean detection time at the second detection point;

It can be shown that the diffusion coefficient of the polymer, for example, is given by:

$$D_{p,n} = \frac{d_c^2\left(t_{d,B} - t_{d,A}\right)}{96\left(\sigma_{B,p,n}^2 - \sigma_{A,p,n}^2\right)} \qquad (33)$$

$$D_{p,n} = \frac{u}{4}\left(H_{p,n} \pm \sqrt{H_{p,n}^2 - \frac{d_c^2}{12}}\right) \qquad (27)$$

where $H_{p,n}$ is given by :

$$H_{p,n} = \left(l_B - l_A\right)\frac{\left(\sigma_{B,p,n}^2 - \sigma_{A,p,n}^2\right)}{\left(t_{d,B} - t_{d,A}\right)^2} \qquad (34)$$

where ($l_B$ - $l_A$) is the capillary length between the two detection points.

## Claims

1. A method for analysing a mixture M comprising:

   (i) a first species which is monodisperse, and
   (ii) a second species which is polydisperse and which has a response coefficient which is distinct from the response coefficient of the first species (i) on at least one detection device,

   said method comprising the following steps:

   (A) the mixture M is injected at the inlet of a capillary tube and forced to be transported in said tube by the flow of a carrier liquid induced by a positive hydrodynamic and/or hydrostatic pressure between the inlet and the outlet of the capillary tube, whereby a phenomenon of Taylor dispersion of the species of the mixture M occurs in the tube;

(B) by using a detector able to detect simultaneously both species (i) and (ii) and placed in the region of the outlet of the capillary tube, a signal reflecting the Taylor dispersion obtained in step (A) is measured;

(C) the signal obtained in step (B) is analysed by using an analysis device, so as to determine specific contributions of species (i) and (ii) and thereby establishing at least one of the followings:

- the content of species (i) and/or (ii) in the mixture M ; and/or
- the mean hydrodynamic radius of the species (ii) or the hydrodynamic radius of species (i),

wherein the species (i) an (ii) have two distinct hydrodynamic radii, and species (i) is a species of a predetermined nature and wherein, in step (C), the respective contributions of species (i) and (ii) in the signal obtained in step (B) are established, whereby the elution profiles of species (i) and (ii) are obtained, which allows to determine the content of species (i) and/or (ii) in the mixture M, and/or the mean hydrodynamic radius of the species (i) and/or (ii), whereby the mixture M is injected so as to make a unit step injection, and the respective contributions of species (i) and (ii) in the signal $T_n(t)$ obtained in step (B) are established, in step (C), by fitting onto the signal $T_n(t)$ the following fit function:

$$C_n(t) = \frac{C_0^i}{2}\left[1 - erf\left(\frac{t - t_{d,n}}{\sigma_{i,n}\sqrt{2}}\right)\right] + \frac{C_0^{ii}}{2}\left[1 - erf\left(\frac{t - t_{d,n}}{\sigma_{ii,n}\sqrt{2}}\right)\right],$$

resulting in the determination of the following parameters: $t_{d,n}$ is the time when an inflexion point is detected in the edge of the signal; $\sigma_{i,n}$ and $\sigma_{ii,n}$, the variances for the contribution of the two species (i) and (ii); and, $C_0^i$ and $C_0^{ii}$, the maximal absorbance of the two species (i) and (ii).

2. The method of claim 1 wherein the mean hydrodynamic radius of the species (ii) is a weight mean, a number mean or a z-mean.

3. The method of any one of claims 1 to 2, wherein the mixture M includes:

- a species (i), non-polymerized molecules of a predetermined nature, and
- a species (ii) macromolecules and/or aggregates and/or particles,

and wherein at least one of the followings is determined in step (C):

- the content of species (i) and/or (ii) in the mixture M; and/or,
- the mean hydrodynamic radius of species (ii).

4. The method according to claim 1, wherein the value of the variance of the elution profile of species (ii) is used to determine a hydrodynamic radius according to the equation:

$$R_{h,ii,n} = \frac{2kT}{3\pi\eta u} \frac{1}{H_{ii,n} \pm \sqrt{H_{ii,n}^2 - \frac{d_c^2}{12}}} \quad \text{with} \quad H_{ii,n} = \frac{l_s.\sigma_{ii,n}^2}{t_d^2}$$

5. The method according to claim 4, wherein the value of the area of the elution profiles of species (i) and (ii) are used

$$\Psi_n = \frac{A_{m,0} - A_{m,n}}{A_{m,0}} .$$

to determine, the degree of conversion $\Psi_n$ according to the equation:

6. The method according to any one of claims 1 to 5, wherein the mixture M is a polymerization medium, and wherein the species (i) is a monomer and the species (ii) are polymers obtained by polymerization of said species (i), wherein the followings are determined in step (C):

- the quantity of monomer (i) in the mixture M, which indicates the degree of conversion of the polymerization; and/or,
- the mean hydrodynamic radius of the polymer (ii).

7. The method according to any one of claims 1 to 6, wherein the mixture M is diluted or dissolved in a medium identical to the carrier liquid used in the Taylor dispersion of step (A), before injecting it in the capillary.

8. The method according to any one of claims 1 to 7, wherein the internal surface of the capillary tube is non-covalently or covalently coated with a compound which limits or inhibits the interaction between the species (i) and (ii) and the inner surface of the capillary tube, said coated compound being chosen from the group consisting in PEO, cellulose derivatives, polyvinyl alcohol, polyacrylamide and its derivatives, polysiloxanes such as polydimethylsiloxane, anionic or cationic (mono- or double-chain) surfactants, polyelectrolyte mono or multilayers.

9. The method according to any one of claims 1 to 8, wherein the mixture M is introduced in the capillary tube together with a carrier liquid including a salt for example NaCl, or a buffer such as phosphate or borate.

10. The method according to any one of claims 1 to 9, wherein a plurality of samples, which each include a mixture of at least two species and having two different response coefficients on at least one detection device, are analysed according to steps (A) to (C) as defined in claim 1 in a same capillary tube, in a sequential way, the samples being injected successively in said capillary tube.

11. An apparatus (1) 1 for a Taylor experiment, comprises a detector (2) and an analysis device (14), said detector comprises a capillary tube (6) through which flows the mixture M to be analysed, injection means (6) for the injection of the mixture M into the capillary tube, and at least one set of optical means (4, 5, 10, 12) to produce a Taylor signal, and said analysis device comprising memorisation means (18), processing means (20) and an I/O interface (16) to receive from said detector said Taylor signal, **characterized in that** said analysis device (14) comprises means for the implementation of an analysis method according to anyone of the claims 1 to 10.

**Patentansprüche**

1. Verfahren zum Analysieren einer Mischung M, umfassend:

   (i) eine erste Spezies, die monodispers ist, und
   (ii) eine zweite Spezies, die polydispers ist und die einen Ansprechkoeffizienten aufweist, der unterschiedlich zu dem Ansprechkoeffizienten der ersten Spezies (i) bei mindestens einer Detektionsvorrichtung ist,

   wobei das Verfahren die folgenden die folgenden Schritte umfasst:

   (A) die Mischung M wird in einen Einlass eines Kapillarrohres eingespritzt und durch den Strom einer Trägerflüssigkeit, die durch einen positiven hydrodynamischen und/oder hydrostatischen Druck zwischen dem Einlass und dem Auslass des Kapillarrohres induziert wird, in diesem Rohr zum Transport gezwungen, wobei ein Phänomen der Taylor-Dispersion der Spezies der Mischung M in dem Rohr auftritt;
   (B) durch Verwendung eines Detektors, der in der Lage ist, gleichzeitig die beiden Spezies (i) und (ii) zu detektieren und in dem Bereich des Auslasses des Kapillarrohres angeordnet ist, wird ein Signal, das die beim Schritt (A) erhaltene Taylor-Dispersion reflektiert, gemessen;
   (C) das in Schritt (B) erhaltene Signal wird unter Verwendung einer Analysevorrichtung analysiert, um so spezifische Beiträge der Spezies (i) und (ii) zu bestimmen und dabei mindestens eines der folgenden herzustellen:

      - den Gehalt der Spezies (i) und/oder (ii) in der Mischung M; und/oder
      - den mittleren hydrodynamischen Radius der Spezies (ii) oder den hydrodynamischen Radius der Spezies (i), wobei die Spezies (i) und (ii) zwei unterschiedliche hydrodynamische Radien aufweisen und Spezies (i) eine Spezies einer vorbestimmten Art ist und wobei in Schritt (C) die jeweiligen Beiträge der Spezies (i) und (ii) in dem bei Schritt (B) erhaltenen Signal erstellt werden, wobei die Elutionsprofile der Spezies (i) und (ii) erhalten werden, die ermöglichen, den Gehalt der Spezies (i) und/oder (ii) in der Mischung M und/oder den mittleren hydrodynamischen Radius der Spezies (i) und/oder (ii) zu bestimmen,

   wobei die Mischung M so eingespritzt wird, dass eine Einschrittinjektion vorgenommen wird und die jeweiligen Beiträge der Spezies (i) und (ii) in dem Signal $T_n(t)$, das in Schritt (B) erhalten wird, in Schritt (C) durch Aufbringen der folgenden Fit-Funktion auf das Signal $T_n(t)$ erstellt werden:

$$C_n(t) = \frac{C_0^i}{2}\left[1 - erf\left(\frac{t - t_{d,n}}{\sigma_{i,n}\sqrt{2}}\right)\right] + \frac{C_0^{ii}}{2}\left[1 - erf\left(\frac{t - t_{c,n}}{\sigma_{ii,n}\sqrt{2}}\right)\right],$$

wodurch sich die Bestimmung der folgenden Parameter ergibt: $t_{d,n}$ ist die Zeit, zu der ein Wendepunkt in der Flanke des Signals detektiert wird; $\sigma_{i,n}$ und $\sigma_{ii,n}$ die Varianzen für den Beitrag der zweit Spezies (i) und (ii) sind; und $C_0^i$ und $C_0^{ii}$ der maximale Absorptionsgrad der zwei Spezies (i) und (ii) sind.

2. Verfahren nach Anspruch 1, bei dem der mittlere hydrodynamische Radius der Spezies (ii) ein Gewichtsmittel, ein Zahlenmittel oder ein z-Mittel ist.

3. Verfahren nach einem der Ansprüche 1 oder 2, wobei die Mischung M einschließt:

    - eine Spezies (i), nichtpolymerisierte Moleküle einer vorbestimmten Art, und
    - eine Spezies (ii) Makromoleküle und/oder Aggregate und/oder Partikel,

und wobei mindestens eines der folgenden in Schritt (C) bestimmt wird:

    - der Gehalt der Spezies (i) und/oder (ii) in der Mischung M; und/oder
    - der mittlere hydrodynamische Radius der Spezies (ii).

4. Verfahren nach Anspruch 1, bei dem der Wert der Varianz des Elutionsprofils der Spezies (ii) verwendet wird, um eine hydrodynamischen Radius entsprechend der Gleichung:

$$R_{h,ii,n} = \frac{2kT}{3\pi\eta u}\frac{1}{H_{ii,n} \pm \sqrt{H_{ii,n}^2 - \frac{d_c^2}{12}}} \text{ mit } H_{ii,n} = \frac{l_s \cdot \sigma_{ii,n}^2}{t_d^2}$$

zu bestimmen.

5. Verfahren nach Anspruch 4, bei dem der Wert der Fläche der Elutionsprofile der Spezies (i) und (ii) verwendet werden, um den Konversionsgrad $\Psi_n$ entsprechend der Gleichung:

$$\Psi_n = \frac{A_{m,0} - A_{m,n}}{A_{m,0}}$$

zu bestimmen.

6. Verfahren nach einem der Ansprüche 1 bis 5, bei dem die Mischung M ein Polymerisationsmedium ist und bei dem die Spezies (i) ein Monomer und die Spezies (ii) Polymere sind, die durch Polymerisation der Spezies (i) erhalten wird, wobei das Folgende in Schritt (C) bestimmt wird:

    - die Menge des Monomers (i) in der Mischung M, die den Konversionsgrad der Polymerisation angibt; und/oder
    - den mittleren hydrodynamischen Radius des Polymers (ii).

7. Verfahren nach einem der Ansprüche 1 bis 6, bei dem die Mischung in einem Medium verdünnt oder gelöst wird, das identisch zu der Trägerflüssigkeit ist, die bei der Taylor-Dispersion des Schritts (A) vor ihrem Einspritzen in die Kapillare verwendet wird.

**8.** Verfahren nach einem der Ansprüche 1 bis 7, bei dem die Innenfläche des Kapillarrohrs nichtkovalent ist oder kovalent mit einer Zusammensetzung beschichtet ist, die die Wechselwirkung zwischen den Spezies (i) und (ii) und der Innenfläche des Kapillarrohrs begrenzt oder sperrt, wobei die beschichtete Verbindung ausgewählt ist aus der Gruppe bestehend aus PEO, Cellulosederivate, Polyvinylalkohol, Polyacrylamid und ihren Derivaten, Polysiloxane, wie Polydimethylsiloxane, anionische oder kationische Tenside (mono- oder doppelkettig), mono oder mehrschichtige Polyelektrolyte.

**9.** Verfahren nach einem der Ansprüche 1 bis 8, bei dem die Mischung M zusammen mit einer Trägerflüssigkeit in das Kapillarrohr eingeführt wird, die ein Salz, beispielsweise NaCl oder einen Puffer, wie ein Phosphat oder Borat, einschließt.

**10.** Verfahren nach einem der Ansprüche 1 bis 9, bei dem eine Mehrzahl von Proben, die jeweils eine Mischung von mindestens zwei Spezies einschließen und zwei unterschiedliche Ansprechkoeffizienten an mindestens einer Detektionsvorrichtung aufweisen, entsprechend den Schritten (A) bis (C), wie im Anspruch 1 definiert, in einem gleichen Kapillarrohr in aufeinanderfolgender Weise analysiert werden, wobei die Proben aufeinanderfolgend in das Kapillarrohr eingespritzt werden.

**11.** Vorrichtung (1) für ein Taylor-Experiment, die einen Detektor (2) und eine Analysevorrichtung (14), wobei der Detektor ein Kapillarrohr (6), durch das die zu analysierende Mischung M strömt, Einspritzmittel (3) für das Einspritzen der Mischung M in das Kapillarrohr und mindestens einen Satz von optischen Mitteln (4, 5, 10, 12), um ein Taylorsignal zu erzeugen, umfasst und die Analysevorrichtung Speichermittel (18), Verarbeitungsmittel (20) und eine I/O-Schnittstelle (16) zum Empfangen des Taylorsignals von dem Detektor umfasst, **dadurch gekennzeichnet, dass** die Analysevorrichtung (14) Mittel zum Implementieren eines Analyseverfahrens nach einem der Ansprüche 1 bis 10 umfasst.

## Revendications

**1.** Procédé pour analyser un mélange M comprenant :

(i) une première espèce qui est monodispersée, et
(ii) une deuxième espèce qui est polydispersée et qui a un coefficient de réponse qui est distinct du coefficient de réponse de la première espèce (i) sur au moins un dispositif de détection,

ledit procédé comprenant les étapes suivantes :

(A) le mélange M est injecté à l'entrée d'un tube capillaire et forcé à être transporté dans ledit tube par le flux d'un liquide porteur induit par une pression hydrostatique et/ou hydrodynamique positive entre l'entrée et la sortie du tube capillaire, en conséquence de quoi un phénomène de dispersion de Taylor de l'espèce du mélange M se produit dans le tube ;
(B) par utilisation d'un détecteur capable de détecter simultanément les deux espèces (i) et (ii) et placé dans la région de la sortie du tube capillaire, un signal reflétant la dispersion de Taylor obtenue dans l'étape (A) est mesuré ;
(C) le signal obtenu dans l'étape (B) est analysé par utilisation d'un dispositif d'analyse, de façon que les contributions spécifiques des espèces (i) et (ii) soient déterminées et de façon à établir ainsi au moins l'un des suivants :

- la teneur en les espèces (i) et/ou (ii) dans le mélange M ; et/ou
- le rayon hydrodynamique moyen de l'espèce (ii) ou le rayon hydrodynamique de l'espèce (i),

dans lequel les espèces (i) et (ii) ont deux rayons hydrodynamiques distincts, et l'espèce (i) est une espèce d'une nature prédéterminée, et dans lequel, dans l'étape (C), les contributions respectives des espèces (i) et (ii) dans le signal obtenu dans l'étape (B) sont établies, en conséquence de quoi les profils d'élution des espèces (i) et (ii) sont obtenus, ce qui permet la détermination de la teneur en les espèces (i) et/ou (ii) dans le mélange M, et/ou le rayon hydrodynamique moyen des espèces (i) et/ou (ii),
en conséquence de quoi le mélange M est injecté de façon que soit effectuée une injection en étape unitaire, et les contributions respectives des espèces (i) et (ii) dans le signal $T_n(t)$ obtenues dans l'étape (B) sont établies, dans l'étape (C), par ajustement sur le signal $T_n(t)$ de la fonction d'ajustement suivante :

$$C_n(t) = \frac{C_0^i}{2}\left[1 - erf\left(\frac{t - t_{d,n}}{\sigma_{i,n}\sqrt{2}}\right)\right] + \frac{C_0^{ii}}{2}\left[1 - erf\left(\frac{t - t_{d,n}}{\sigma_{ii,n}\sqrt{2}}\right)\right],$$

ayant pour résultat la détermination des paramètres suivants : $t_{d,n}$ est le moment où un point d'inflexion est déterminé dans le bord du signal ; $\sigma_{i,n}$ et $\sigma_{ii,n}$ sont les variances pour la contribution des deux espèces (i) et (ii) ; et $C_0^i$ et $C_0^{ii}$ représentent l'absorbance maximale des deux espèces (i) et (ii).

2. Procédé selon la revendication 1, dans lequel le rayon hydrodynamique moyen de l'espèce (ii) est une moyenne en poids, une moyenne en nombre ou une moyenne en z.

3. Procédé selon l'une quelconque des revendications 1 et 2, dans lequel le mélange M contient :

   - une espèce (i) constituée de molécules non polymérisées de nature prédéterminée, et
   - une espèce (ii) constituée de macromolécules et/ou d'agrégats et/ou de particules, et dans lequel au moins l'un des suivants est déterminé dans l'étape (C) :
   - la teneur en les espèces (i) et/ou (ii) dans le mélange M ; et/ou
   - le rayon hydrodynamique moyen de l'espèce (ii).

4. Procédé selon la revendication 1, dans lequel la valeur de la variante du profil d'élution de l'espèce (ii) est utilisée pour la détermination d'un rayon hydrodynamique conformément à l'équation :

$$R_{h,ii,n} = \frac{2kT}{3\pi\eta u}\frac{1}{H_{ii,n} \pm \sqrt{H_{ii,n}^2 - \frac{d_c^2}{12}}} \quad \text{avec} \quad H_{ii,n} = \frac{l_s.\sigma_{ii,n}^2}{t_d^2}$$

5. Procédé selon la revendication 4, dans lequel la valeur de la superficie des profils d'élution des espèces (i) et (ii) est utilisée pour la détermination du degré de conversion $\psi_n$ conformément à l'équation :

$$\psi_n = \frac{A_{m,0} - A_{m,n}}{A_{m,0}}$$

6. Procédé selon l'une quelconque des revendications 1 à 5, dans lequel le mélange M est un milieu de polymérisation, et dans lequel l'espèce (i) est un monomère et l'espèce (ii) est constituée de polymères obtenus par polymérisation de ladite espèce (i), et dans lequel les points suivants sont déterminés dans l'étape (C) :

   - la quantité de monomère (i) dans le mélange M, qui indique le degré de conversion de la polymérisation ; et/ou
   - le rayon hydrodynamique moyen du polymère (ii).

7. Procédé selon l'une quelconque des revendications 1 à 6, dans lequel le mélange M est dilué ou dissous dans un milieu identique au liquide porteur utilisé dans la dispersion de Taylor dans l'étape (A), avant son injection dans le capillaire.

8. Procédé selon l'une quelconque des revendications 1 à 7, dans lequel la surface interne du tube capillaire est revêtue, de manière covalente ou non covalente, par un composé qui limite ou inhibe l'interaction entre les espèces (i) et (ii) et la surface intérieure du tube capillaire, ledit composé déposé sous forme de revêtement étant choisi dans le groupe constitué par le PEO, les dérivés de cellulose, le poly(alcool vinylique), le polyacrylamide et ses dérivés, les polysiloxanes tels que le polydiméthylsiloxane, les tensioactifs anioniques ou cationiques (à chaîne simple ou double), les monocouches ou multicouches de polyélectrolytes.

**9.** Procédé selon l'une quelconque des revendications 1 à 8, dans lequel le mélange M est introduit dans le tube capillaire conjointement avec un liquide porteur contenant un sel, par exemple NaCI, ou un tampon tel qu'un phosphate ou borate.

**10.** Procédé selon l'une quelconque des revendications 1 à 9, dans lequel une pluralité d'échantillons, qui contiennent chacun un mélange d'au moins deux espèces et ont deux coefficients de réponse différents sur au moins un dispositif de détection, sont analysés conformément aux étapes (A) à (C) telles que définies dans la revendication 1 dans un même tube capillaire, de manière séquentielle, les échantillons étant injectés successivement dans ledit tube capillaire.

**11.** Dispositif (1) 1 pour une expérience de Taylor, comprenant un détecteur (2) et un dispositif d'analyse (14), ledit détecteur comprenant un tube capillaire (6) dans lequel s'écoule le mélange M devant être analysé, des moyens d'injection (6) pour l'injection du mélange M dans le tube capillaire, et au moins un jeu de moyens optiques (4, 5, 10, 12) pour produire un signal de Taylor, ledit dispositif d'analyse comprenant des moyens de mémorisation (18), des moyens de traitement (20) et une interface d'entrée/sortie (16) pour recevoir ledit signal de Taylor à partir dudit détecteur, **caractérisé en ce que** ledit dispositif d'analyse (14) comprend des moyens pour la mise en oeuvre d'un procédé d'analyse selon l'une quelconque des revendications 1 à 10.

FIG.1

$$Fit \; T_n(t)$$

$$\sigma_{m,n}^2 \quad C_o^m$$
$$\sigma_{p,n} \quad C_o^p$$
$$t_{d,n}$$

$k_m \, AND \; k_p \; known$
$AND$
$k_m \neq k_p$
$?$

N

Y

2

$S_{m,n}(t)$

$S_{p,n}(t)$

N

Y

2.2

Reduction of the number of parameters $t_{d,n} \quad \sigma_{m,n}$

Fit $S_n(t)$

$S_n'(t) = S_n(t) - S_{m,n}(t)$

2.1

Reduction of the number of parameters $t_{d,n} \quad \sigma_{m,n}$

Fit $S_n(t)$

$m_n$ constant?

N

Y

1

1.2

$A_0 \, h_0 \, \sigma_m$

$A_n \, h_n \, \sigma_n$

$$\alpha_n = \frac{h_n}{h_0} \frac{A_0}{A_n} \; ; \quad \beta_n = \frac{\sigma_n^2}{\sigma_m^2}$$

1.1

$A_0$

$A_n$

## FIG.2 beginning

25

$$x_n = \frac{1}{2}\left(\sqrt{\frac{4\beta_n - 3 - \alpha_n}{1 - \alpha_n}} - 1\right)$$

$$k = \frac{k_p}{k_m}$$

$$A_{p,n} \quad A_{m,n} \quad \sigma^2_{p,n}$$

$$\sigma^2_{p,n}$$

$$D_{m,n} \quad R_{h,m,n}$$
$$D_{p,n} \quad R_{h,p,n}$$

$$\Psi_n = \frac{1}{1-\kappa}\frac{A_0 - A_n}{A_0}$$

$$\Psi_n = \frac{y_n}{y_n + \kappa(1 - y_n)}$$

$$\Psi_n = \frac{A_{m,0} - A_{m,n}}{A_{m,0}}$$

$$H_{p,n} \quad D_{p,n} \quad R_{h,p,n}$$

# FIG.2 end

EP 2 313 768 B1

FIG.3

FIG.4

## FIG.5

## FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **COTTET H et al.** Taylor dispersion analysis of mixtures. *NALYTICAL CHEMISTRY,* 01 December 2002, vol. 79 (23), 9066-9073
- **KELLY B et al.** Using Taylor dispersion profiles to characterize polymer molecular weight distributions. *PCCP - PHYSICAL CHEMISTRY CHEMICAL PHYSICS,* 21 December 2004, vol. 6 (24), ISSN 1 463-9076, 5523-5530
- **CALLENDAR R et al.** Diffusion coefficients for binary, ternary, and polydisperse solutions from peak-width analysis of Taylor dispersion profiles. *JOURNAL OF SOLUTION CHEMISTRY,* March 2006, vol. 35 (3), ISSN 0095-9782, 353-379
- **G. TAYLOR.** *Proc. Roy. Soc., A,* 1953, vol. 219, 186-203
- **R. ARIS.** *Proc. Roy. Soc. Lond. A.,* 1956, vol. 235, 67-77
- **H. COTTET ; M. MARTIN ; A. PAPILLAUD ; E. SOUAÏD ; H. COLLET ; A. COMMEYRAS.** *Biomacromolecules,* 2007, vol. 8, 3235-3243
- **A. J. S. CHAPMAN ; D. M. GOODHALL.** *Chromatography Today,* June 2008, vol. 1, 22-24
- *J. Phys. Chem.,* 1974, vol. 78, 2297-2301
- *Science,* 1994, vol. 266, 773-776